# EUROPEAN PATENT APPLICATION

(11) **EP 4 238 585 A1**
(43) Date of publication of application: **06.09.2023**
(21) Application number: 21900430.6
(22) Date of filing: 19.11.2021
(51) Int. Cl.: A61L 2/10, B01J 19/12, A61L 9/20

(54) **ULTRAVIOLET RAY IRRADIATION DEVICE AND ULTRAVIOLET RAY IRRADIATION METHOD**

(30) Priority: 01.12.2020 JP 2020199935; 14.01.2021 JP 2021004239
(71) Applicant: Ushio Denki Kabushiki Kaisha, Tokyo 100-8150 (JP)
(72) Inventor: NAITO, Keisuke, Tokyo 100-8150 (JP); TERADA, Shoichi, Tokyo 100-8150 (JP); SABATAKE, Kenichi, Tokyo 100-8150 (JP)
(74) Representative: Tomerius, Isabel
(86) International application number: PCT/JP2021/042536
(87) International publication number: WO 2022/118675

(57) **Abstract**

Provided is an ultraviolet light irradiation device and an ultraviolet light irradiation method that can effectively and more appropriately perform the inactivation of microorganisms and/or viruses using ultraviolet light having a wavelength range in which adverse effects on human bodies are suppressed. The ultraviolet light irradiation device includes a light source section having a light emission surface emitting ultraviolet light having a wavelength band from 190 nm to 235 nm, a controller that controls lighting of the light source section, and a distance sensor that measures a separation distance from the light emission surface to an object facing the light emission surface. The controller is provided with a plurality of lighting operation modes. Each of the plurality of lighting operation modes is determined corresponding to a section defined by the separation distance, and is set such that an amount of the ultraviolet light emitted from the light source section differs from each other. The controller controls to switch the lighting operation mode based on a signal from the distance sensor.

## Description

### TECHNICAL FIELD

The present invention relates to an ultraviolet light irradiation device and an ultraviolet light irradiation method for inactivating harmful microorganisms and/or viruses by irradiating them with ultraviolet light.

### BACKGROUND ART

Microorganisms (bacteria, fungi, etc.) and viruses present in a space or on the front surface of objects can cause infectious diseases in humans and non-human animals, and there is concern that the spread of infectious diseases may threaten people's lives. In particular, infectious diseases are more likely to spread in facilities such as medical facilities, schools, and government offices, and in places where people frequently gather or where people heavily come and go, such as automobiles, trains, buses, airplanes, ships, and other vehicles.

Patent document 1 discloses a technique for inactivating bacteria while substantially avoiding harm to the cells of human or animal bodies. This Patent document 1 describes that ultraviolet light disinfection can be used to break down microorganisms in food, air, and drinking water, typically using the ultraviolet light of UVB or UVC, and that this ultraviolet light is harmful to humans and other living organisms. It further states that ultraviolet light having a wavelength of above 240 nm causes damage to DNA in the nuclei of human cells, and that the penetrating power of ultraviolet lightvaries with its wavelength; radiation having shorter wavelengths has less penetrating power, resulting in less harmfulness to human cells.

### CITATION LIST

Patent Document 1: Japanese Patent No. 6025756

### SUMMARY OF THE INVENTION

### Technical Problem

In accordance with Patent Document 1, ultraviolet light having a wavelength band from 190nm to 235 nm, which is of controlled harmfulness to humans and animals, has been examined to disinfect at least one bacteria present on or in humans. According to the American Conference of Governmental Industrial Hygienists (ACGIH) and JIS Z 8812 (Measuring Methods of Eye-hazardous Ultraviolet Radiation), the amount of ultraviolet light irradiation per day (8 hours) to human bodies is specified as a threshold limit value (TLV) on each wavelength, thereby requiring the amount of illuminance and irradiation of ultraviolet light radiated per a given period to be determined to the extent that they do not exceed the threshold limit value. Although this threshold limit value may be revised in the future, it is preferable to set a certain upper limit on the amount of ultraviolet light irradiation for safer operation.

Meanwhile, ultraviolet light having a wavelength from 190 nm to 235 nm diffuses in the atmosphere, and its illuminance decreases inversely proportional to the square of the distance. In other words, the illuminance value varies greatly with the separation distance from the light source. Hence, in the case of setting the ultraviolet light illuminance suitable for inactivating microorganisms and/or viruses to a given target space or the surface of a target object, it is necessary to emit ultraviolet light from the light source with a higher illuminance.

Accordingly, for more effective inactivation at different separation distances from the light source, it is desirable to change the cumulative amount of radiation and irradiation intensity of ultraviolet light according to the separation distance from the light source to the object to be inactivated. In particular, it is desirable to control them appropriately in situations where objects, including people, are moving in an environment where microorganisms and/or viruses are intended to be inactivated.

It is an object of the present invention to provide an ultraviolet light irradiation device and an ultraviolet light irradiation method that can effectively and more appropriately perform the inactivation of microorganisms and/or viruses using ultraviolet light having a wavelength range in which adverse effects on human bodies is suppressed.

### Solution to the Problem

To solve the above-mentioned problem, an aspect of the ultraviolet light irradiation device according to the present invention includes: a light source section having a light emission surface emitting ultraviolet light having a wavelength band from 190 nm to 235 nm; a controller that controls lighting of the light source section; and a distance sensor that measures a separation distance from the light emission surface to an object facing the light emission surface. The controller is provided with a plurality of lighting operation modes. Each of the plurality of lighting operation modes is determined corresponding to a section defined by the separation distance, and is set such that an amount of the ultraviolet light emitted from the light source section differs from each other. The controller controls to switch the lighting operation mode based on a signal from the distance sensor.

As described above, radiating ultraviolet light having a wavelength range from 190 nm to 235 nm, which has less adverse effects on human and animal cells, enables the ultraviolet light irradiation for inactivating bacteria and viruses even in a space where people are present. In addition, by controlling to switch the lighting operation mode based on the section defined by the separation distance from the light emission surface, the amount of ultraviolet light radiated can be changed to an appropriate amount based on the value of the separation distance. In addition, the plurality of lighting operation modes are defined corresponding to the sections defined by the respective separation distances from the light-emitting surface, and the switching of lighting operation mode is executed when the section (distance range) defined by the separation distance from the light-emitting surface is shifted. This allows the same lighting operation mode to be executed when an object exists in the predetermined section (distance range), thereby preventing the lighting operation modes from being switched when the separation distance varies slightly. Hence, even in an environment where an object moves in the space where ultraviolet light radiates, the lighting operation mode can be switched appropriately, thereby preventing malfunction of the device.

In the above-mentioned ultraviolet light irradiation device, the plurality of lighting operation modes may be set such that the amount of the ultraviolet light emitted from the light source section becomes lower in the section to which the separation distance from the light emission surface is relatively closer. This allows the amount of ultraviolet light irradiation to be appropriately controlled in accordance with the value of the separation distance from the light source.

In the above-mentioned ultraviolet light irradiation device, the plurality of lighting operation modes may be set such that the illuminance of the ultraviolet light emitted from the light source section at the light emission surface becomes lower in the section to which the separation distance from the light emission surface is relatively closer. Specifically, when section A where the separation distance from the light emission surface is relatively close is compared with section B where the separation distance from the light emission surface is far, the lighting operation mode of section A has a reduced illuminance of the ultraviolet light emitted from the light source section at the light emission surface than the lighting operation mode of section B. This allows the amount of ultraviolet light irradiation to be appropriately controlled in accordance with the separation distance from the light source.

In the above-mentioned ultraviolet light irradiation device, the plurality of lighting operation modes may control the lighting of the light source section such that a lighting duty ratio of the light source section is reduced in the section to which the separation distance from the light emission surface is relatively closer. This allows the amount of ultraviolet light irradiation to be appropriately controlled in accordance with the value of the separation distance from the light source.

In addition, the above-mentioned ultraviolet light irradiation device may further include a human detection sensor that detects a location of a person facing the light emission surface, and the controller may control to switch the lighting operation mode based on a signal from the distance sensor when a presence of a person is detected by the human detection sensor. In this case, the lighting operation mode can be switched appropriately even in an environment where a person moves in a space where ultraviolet light radiates. In addition, setting the amount of the ultraviolet light radiated from the light source section to decrease as a person approaches closer to the light emission surface makes it possible to appropriately suppress the irradiation of a high energy amount of light to a person located at a close distance to the light source.

In the above-mentioned ultraviolet light irradiation device, the controller may be set such that when the presence of a person is undetected by the human detection sensor, the amount of ultraviolet light emitted from the light source section becomes higher than that when the presence of a person is detected by the human detection sensor. This enables the inactivation of microorganisms and/or viruses to be effectively performed.

The above-mentioned ultraviolet light irradiation device may further include a drive unit that changes an orientation of the light emission surface, and the distance sensor may measure the separation distance to an object facing the light emission surface corresponding to the orientation of the light emission surface. This can appropriately measure the separation distance from the light emission surface to an object, and control to switch to a suitable lighting operation mode, even when the direction of the ultraviolet light radiation is changed.

Furthermore, an aspect of the ultraviolet light irradiation method according to the present invention is an ultraviolet light irradiation method of controlling lighting of a light source section having a light emission surface emitting ultraviolet light having a wavelength band from 190 nm to 235 nm, and includes the steps of: receiving a signal from a distance sensor measuring a separation distance to an object facing the light emission surface; and switching a plurality of lighting operation modes to control the lighting of the light source section based on the signal from the distance sensor, each of the plurality of lighting operation modes being determined corresponding to a section defined by the separation distance from the light emission surface, and being set in a manner that an amount of the ultraviolet light emitted from the light source section is different from each other.

In this way, radiating ultraviolet light having a wavelength range from 190 nm to 235 nm, which has less adverse effects on human and animal cells, enables the ultraviolet light even in spaces where people are present, thereby capable of inactivating bacteria and viruses. In addition, by controlling to switch the lighting operation mode in accordance with the section defined by the separation distance from the light emission surface, the amount of radiated ultraviolet light can be changed to an appropriate amount in accordance with the value of the separation distance. The plurality of lighting operation modes is defined as corresponding to sections defined by the separation distance from the light emission surface, and the switching of lighting operation mode is executed when the section (distance range) defined by the separation distance from the light-emitting surface is shifted. This allows the same lighting operation mode to be executed when an object is present within a predetermined section (distance range), thereby eliminating the execution of the switching of lighting operation mode when the separation distance changes slightly. Hence, the lighting operation mode can be switched appropriately even in an environment where an object moves in a space where ultraviolet light radiates, preventing the device from malfunctioning.

### Effects of the Invention

An aspect of the present invention is capable of inactivating microorganisms and/or viruses effectively and more appropriately using ultraviolet light having a wavelength range with less adverse effects on human bodies.

### BRIEF DESCRIPTION OF DRAWINGS

FIG. 1 is an appearance view of an ultraviolet light irradiation device according to the present embodiment.
FIG. 2 is a conceptual diagram illustrating sections of a separation distance from an ultraviolet light irradiation device.
FIG. 3 is an example of an operation in a case where a lighting duty ratio is reduced by changing unlit time.
FIG. 4 is an example of an operation in a case where the lighting duty ratio is reduced by changing lighting time.
FIG. 5 is an example of an operation in a case where the illuminance of intermittent lighting is reduced.
FIG. 6 is an example of an operation in a case where the illuminance of continuous lighting is reduced.
FIG. 7 is a schematic view of an ultraviolet light irradiation device in which the direction of ultraviolet light irradiation is variable.
FIG. 8A to 8D are examples of operation in a case of changing the direction of ultraviolet light irradiation.

### DESCRIPTION OF EMBODIMENTS

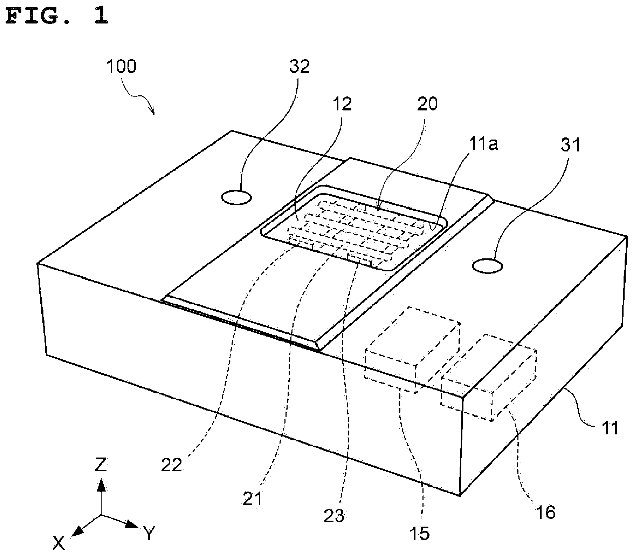

Hereinafter, embodiments of the present invention will be described with reference to the drawings. FIG. 1 is an appearance view of an ultraviolet light irradiation device 100 according to the present embodiment. The ultraviolet light irradiation device 100 is a device that performs ultraviolet light irradiation in a space where people or animals are present to inactivate microorganisms and/or viruses present in the space or on a surface of objects in the space. Here, the above space includes, for example, a space in offices, commercial facilities, medical facilities, station facilities, schools, government offices, theaters, hotels, restaurants, or other facilities, or a space in automobiles, trains, buses, cabs, planes, ships, or other vehicles. The above spaces may be enclosed spaces such as hospital rooms, conference rooms, restrooms, and elevators, or may not be enclosed spaces.

The ultraviolet light irradiation device 100 irradiates a space with ultraviolet light having a wavelength range from 190 nm to 235 nm (more preferably, a wavelength range from 200 nm to 230 nm), which has few adverse effects on human and animal cells, thereby inactivating harmful microorganisms and/or viruses present on the surface of objects in the target space and in the space. Here, the above objects include human bodies, animals, and things. The target space to be irradiated with ultraviolet light is not limited to spaces where people or animals are actually present; however, it also includes spaces where people or animals enter and leave and spaces where no people or animals are present. The term "inactivation" here refers to eliminating microorganisms and/or viruses (or causing them to lose their infectivity or toxicity).

As shown in FIG. 1, the ultraviolet light irradiation device 100 includes a light source section that generates ultraviolet light, a controller 16 that controls the lighting of the light source section, and a housing 11 that houses the light source section and the controller 16. The housing 11 has a light emission surface 12 through which ultraviolet light radiates. Specifically, the housing 11 has an opening 11a that serves as a light emission window through which ultraviolet light radiates. This opening 11a is provided with a window member made of quartz glass, for example, and ultraviolet light is emitted from the window member. The opening 11a can also be provided with other components such as an optical filter that blocks light with unnecessary wavelength bands. In addition, the ultraviolet light irradiation device 100 also includes a detector 31 that detects the presence of a person and a distance sensor 32 that measures a separation distance to an object facing the light emission surface 12. An excimer lamp 20 is housed inside the housing 11 as an ultraviolet light source. The excimer lamp 20 can be, for example, a KrCI excimer lamp that emits ultraviolet light having a center wavelength of 222 nm. The ultraviolet light source is not limited to a KrCI excimer lamp, but can be any light source that emits ultraviolet light in the wavelength range from 190 nm to 235 nm. The housing 11 and the ultraviolet light source (excimer lamp 20) constitute the light source section.

The penetrating power of UV radiation varies with wavelength; light with shorter wavelengths has less penetrating power. For example, UV radiation having a short wavelength, such as about 200 nm, passes through water very efficiently, but is absorbed by the outer parts of human cells (cytoplasm) and may not have enough energy to reach the cell nuclei containing DNA that is sensitive to UV radiation. Hence, the above short-wavelength UV radiation has few adverse effects on human cells. In contrast, ultraviolet light having a wavelength of above 240 nm can damage DNA in human cell nuclei. In addition, ultraviolet light with a wavelength of less than 190 nm is known to generate ozone. Hence, in the present embodiment, used is an ultraviolet light source that emits ultraviolet light having a wavelength band from 190 nm to 235 nm, which provides an inactivation effect with few adverse effects on human bodies and, and that emits substantially no UVC except the above wavelength band. In addition, an ultraviolet light source having a peak wavelength in the wavelength range from 200 nm to 230 nm may be used as an even safer wavelength band.

The excimer lamp 20 is provided with a straight-tube discharge container 21 that is hermetically sealed at its both ends. The discharge container 21 can be made of quartz glass, for example. The inside of the discharge container 21 is filled with a noble gas and halogen as an emission gas. In the present embodiment, a krypton chloride (KrCI) gas is used as the emission gas. In this case, the peak wavelength of light emitted is 222 nm. The emission gas is not limited to the above; a krypton bromide (KrBr) gas can also be used as the emission gas. In the case of a KrBr excimer lamp, the peak wavelength of light emitted is 207 nm. In FIG. 1, the ultraviolet light irradiation device 100 includes the plurality of (three) discharge containers 21; however, the number of discharge containers 21 can be any number.

A pair of electrodes (a first electrode 22 and a second electrode 23) are arranged to be in contact with the outer front surface of the discharge containers 21. The first electrode 22 and the second electrode 23 are arranged on the opposite surface (surface in the-Z direction) with respect to a light extraction surface in the discharge container 21, and are spaced apart from each other in the tube axial direction (the Y direction) of the discharge container 21. The discharge containers 21 are then arranged in a manner that they straddle these two electrodes 22 and 22 while being in contact with them. Specifically, the two electrodes 22 and 23 have concave grooves formed therein, and allow the discharge containers 21 to be fitted into the concave grooves of the electrodes 22 and 23.

Of this pair of electrodes, one electrode (e.g., the first electrode 22) is a high-voltage electrode and the other electrode (e.g., the second electrode 23) is a low-voltage electrode (ground electrode). The lamp is lit by applying a high-frequency voltage between the first electrode 22 and the second electrode 23.

The light extraction surface of the excimer lamp 20 is disposed opposite the light emission window. Hence, light emitted from the excimer lamp 20 is emitted from the light emission surface 12 of the ultraviolet light irradiation device 100 through the light emission window. Here, the electrodes 22 and 23 may be made of a metal that has reflectivity to the light emitted from the excimer lamp 21. This configuration allows the light emitted from the discharge container 21 in the -Z direction to be reflected and travel in the +Z direction.

The opening 11a, which serves as a light emission window, can be provided with an optical filter as described above. The optical filter can be a wavelength selection filter that transmits light having a wavelength range of 190 nm to 235nm, which is less adverse effects on human bodies, (more preferably, light having a wavelength range of 200 nm to 230 nm), and blocks UVC light having a wavelength range from 236 nm to 280nm. Specifically, of the illuminance of the peak wavelength of ultraviolet light in the wavelength band from 190 nm to 235 nm, the optical filter can reduce the illuminance of ultraviolet light in each wavelength from 236 nm to 280 nm to 1% or less. Examples of the wavelength selection filter include an optical filter having a dielectric multilayer film consisting of HfO₂ layers and SiO₂ layers.

Examples of the wavelength selection filter also include an optical filter having a dielectric multilayer film consisting of SiO₂ layers and Al₂O₃ layers. Hence, providing the optical filter in the light emission window can reliably suppress the light from leaking out of the housing 11 even when light harmful to humans is emitted from the excimer lamp 20.

As shown in FIG.1, the ultraviolet light irradiation device 100 also includes a power supply section 15 and the controller 16. The power supply section 15 includes power supply components such as an inverter to which power is supplied from a power source, and cooling components such as a heat sink to cool the power supply components. The controller 16 controls the lighting of the excimer lamp 20 that constitutes the light source section.

The detector 31 can be a human detection sensor that detects a person present in an area (irradiation area) irradiated with ultraviolet light emitted from the light emission surface 12. Examples of the human sensor include a pyroelectric infrared sensor that detects changes in heat (infrared radiation) emitted from human bodies and the like. When the detector 31 detects the presence of a person, it transmits a detection signal to the controller 16.

The distance sensor 32 detects a separation distance from the light emission surface 12 to a target object in a direction orthogonal to the light emission surface 12. Here, the target object includes a person, an animal, and an object. The distance sensor 32 can be an infrared sensor that includes, for example, an infrared light-emitting element such as an infrared LED and a light-receiving element such as a photodiode, and that detects the distance to the object by receiving infrared light emitted from the infrared light-emitting element and reflected by the object with the light-receiving element. When the distance sensor 32 detects an object facing the light emission surface 12, it transmits a detection signal to the controller 16. The distance sensor 32 is not limited to the infrared sensor described above; it can be any sensor as long as it can measure the separation distance from the object facing the light emission surface 12.

The controller 16 is provided with a plurality of lighting operation modes. Each lighting operation mode is determined corresponding to a section defined by the separation distance from the light emission surface 12, and is set such that the amount of ultraviolet light emitted from the light source section (the amount of ultraviolet light radiation) differs from each other. The controller 16 controls to switch the lighting operation mode based on the signal from the distance sensor 32.

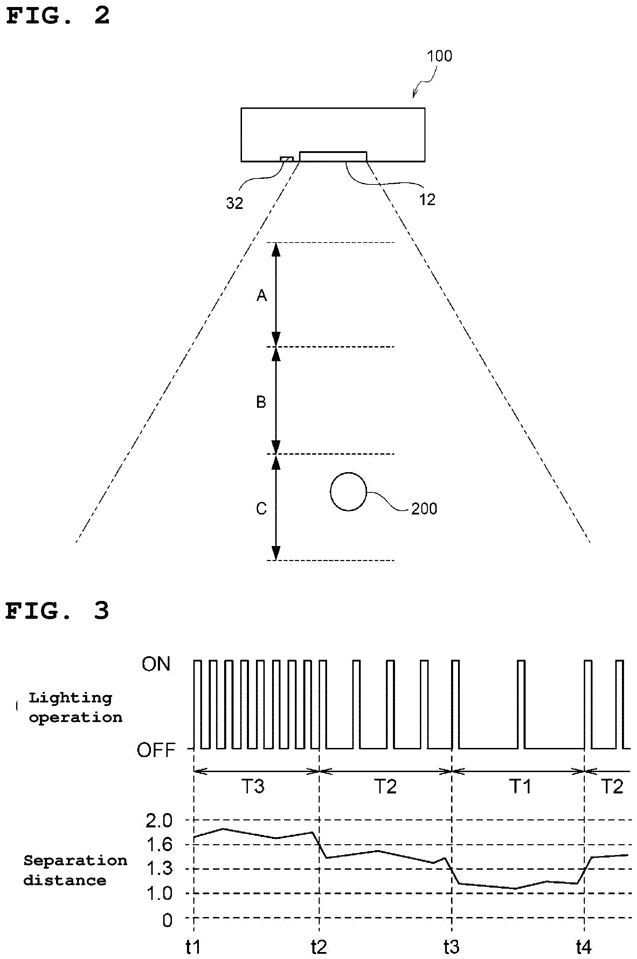

FIG. 2 is a conceptual diagram illustrating sections (distance ranges) defined by the separation distance from the light irradiation surface 12, and shows the relationship between sections A, B, and C and an object 200 detected by the distance sensor 32 as an aspect. The plurality of lighting operation modes provided in the controller 16 is determined corresponding to the sections (section A, section B, section C) defined by the separation distance from the light irradiation surface 12, and the lighting operation modes different from each other are set for the respective sections. This allows the lighting operation mode of the light source section to be switched depending on the section in which the object 200 is present.

The controller 16 also sets a lighting operation mode in which the amount of ultraviolet light emitted from the light source section is reduced in the section to which the separation distance from the light emission surface 12 is relatively close, compared with the section to which the separation distance is relatively far. For example, when the object 200 is determined to be present in the section A, the lighting operation mode is set such that the lighting time per predetermined time is controlled to be shorter, the illuminance of ultraviolet light at the light emission surface 12 is reduced, or the lighting duty ratio (lighting time per lighting period) is reduced in the case of so-called intermittent lighting, which undergoes a periodic on/off lighting operation, compared with the case where the object 200 is determined to be present in the section C. This enables the difference in the amount of ultraviolet light radiated to the object 200 between the case where the object 200 is present in the section A and the case where the object 200 is present in the section C to be controlled smaller. Furthermore, when the object 200 is a person, this enables the control such that the amount of ultraviolet light radiated to the object 200 does not exceed the ACGIH threshold limit value.

In addition, the ultraviolet light irradiation device 100 may include a human detection sensor as the detector 31, as described above. This makes it possible to detect whether or not a person is present in the space where ultraviolet light radiates. This allows the operation pattern of the lighting operation mode to be changed between the case where a person is present and the case where no person is present (the absence of a person). This enables the control of the operation pattern in which the ACGIH threshold limit values are considered in the case where a person is present.

The following exemplifies several embodiments of the ultraviolet light irradiation device according to the present invention. In the first embodiment, the separation distance between the light emission surface 12 and the object 200 is determined based on the signal from the distance sensor 32. In addition, four sections are set in terms of the separation distance from the light emission surface 12, and the lighting operation modes (operation modes 1 to 4) different from each other are set for the respective sections. Table 1 shows the specific lighting operations.

**Table 1**

| Name | Separation distance from light emission surface | Lighting operation pattern | |
|---|---|---|---|
| | Section (Distance range) [m] | Lighting time [second] | Non-lighting time [second] |
| Operation mode 1 | 1.0 - 1.29 | 15 | 345 |
| Operation mode 2 | 1.3 - 1.59 | 15 | 200 |
| Operation mode 3 | 1.6 - 1.99 | 15 | 125 |
| Operation mode 4 | 2.0 or more | 15 | 75 |

As shown in Table 1, the operating modes 1 to 4 are set such that the operating mode 4 is used when the object 200 is present in the distance, and the non-lighting time is increased when the object including a person is present in a section closer to the light emission surface 12. When the object 200 is detected in a relatively closer section, the lighting operation pattern is changed to reduce the lighting duty ratio. For example, when the object 200 is determined to be present at a separation distance of 1.4 m from the light emission surface 12, the operation mode 2 is selected to execute a lighting operation pattern of 15 seconds on and 200 seconds off. If the object 200 is determined to be present at a separation distance of 1.2 m, the operation mode 2 is selected to execute a lighting operation pattern of 15 seconds on and 345 seconds off. Since it is assumed that the object 200 facing the light emission surface 12 is a person, the lighting operation pattern is preferably set such that the amount of ultraviolet light irradiation to the object 200 does not exceed the ACGIH threshold limit value (TLV).

In the second embodiment, the lighting operation pattern is controlled to be switched by using the detector (human detection sensor) 31 together with the distance sensor 32. Four sections are set in terms of the separation distance from the light emission surface 12, and the lighting operation modes (operation modes 0 to 4) different from each other are set for the respective sections. Table 2 shows the specific lighting operations.

**Table 2**

| Name | Separation distance from light emission surface | Lighting operation pattern | |
|---|---|---|---|
| | Section (Distance range) [m] | Lighting time [second] | Non-lighting time [second] |
| Operation mode 0 | - | 15 | 30 |
| Operation mode 1 | 1.0 - 1.29 | 15 | 345 |
| Operation mode 2 | 1.3 - 1.59 | 15 | 200 |
| Operation mode 3 | 1.6 - 1.99 | 15 | 125 |
| Operation mode 4 | 2.0 or more | 15 | 75 |

In the second embodiment, if the presence of a person is determined to be undetected by the human detection sensor 31 (i.e. when a person is absent), the operation mode 0 is set to continue the predetermined lighting operation pattern as a basic operation. When the presence of a person is detected by the human detection sensor 31, the operation modes 1 to 4 are set. The operation modes 1 to 4 correspond to the respective four sections defined by a separation distance from the light emission surface 12. When the object 200 is determined to be present in a given section based on the signal from the distance sensor 32, the operation mode corresponding to that section is selected. For example, if the object 200 is determined to be present at a separation distance of 1.4 m from the light emission surface 12, the operation mode 2 is selected to execute the lighting operation pattern of 15 seconds on and 200 seconds off. If the object 200 is determined to be present at a separation distance of 1.2 m, the operation mode 1 is controlled to be switched to execute the lighting operation pattern of 15 seconds on and 345 seconds off.

In the above-mentioned embodiments, the non-lighting time is changed in the periodic lighting/non-lighting cycle as the lighting operation patterns. FIG. 3 is a timing chart illustrating an example of an operation in which the lighting duty ratio is changed by changing the non-lighting time of intermittent lighting based on the signal from the distance sensor 32. FIG. 3 shows a conceptual diagram, and the lighting (ON) time and the non-lighting (OFF) time are different from those shown in the above-mentioned Tables 1 and 2.

When a person moves in the space where ultraviolet light radiates, the controller 16 in the ultraviolet light irradiation device 10 uses the signal from the distance sensor 32 to measure the separation distance between the person and the light emission surface 12. Then, the controller 16 sets the period (time t1 to t2) during which a person is present in a separation distance of 1.6 m to 1.99 m from the light emission surface 12 to be a period T3 for selecting the operation mode 3, and executes the lighting operation pattern for the operation mode 3 in Table 2. When a person moves in a range of 1.3 m to 1.59 m, the controller 16 sets the period (time t2 to t3) during which a person is present in a separation distance of 1.3 m to 1.59 m from the light emission surface 12 to be a period T2 for selecting the operation mode 2, based on the signal from the distance sensor 32. Then, during this period T2, the controller 16 reduces the lighting duty ratio of intermittent lighting by lengthening the non-lighting time than that of the period T3. In other words, the amount of ultraviolet light emitted from the light source section is lowered. When a person moves in a range of 1.0 m to 1.29 m, the controller 16 sets the period (time t3 to t4) during which a person is present in a separation distance of 1.0 m to 1.29 m from the light emission surface 12 to be a period T1 for selecting the operation mode 1, based on the signal from the distance sensor 32. Then, during this period T1, the controller 16 reduces the lighting duty ratio of intermittent lighting by lengthening the non-lighting time longer than that of the period T2. In other words, the amount of ultraviolet light emitted from the light source section is further lowered.

The operation patterns applicable to the present invention are not limited to the above embodiments. For example, the lighting duty ratio may be variably controlled by changing the lighting time. Table 3 shows the lighting operation in this case.

**Table 3**

| Name | Separation distance from light emission surface | Lighting operation pattern | |
|---|---|---|---|
| | Section (Distance range) [m] | Lighting time [second] | Non-lighting time [second] |
| Operation mode 1 | 1.0 - 1.29 | 10 | 300 |
| Operation mode 2 | 1.3 - 1.59 | 20 | 300 |
| Operation mode 3 | 1.6 - 1.99 | 35 | 300 |
| Operation mode 4 | 2.0 or more | 55 | 300 |

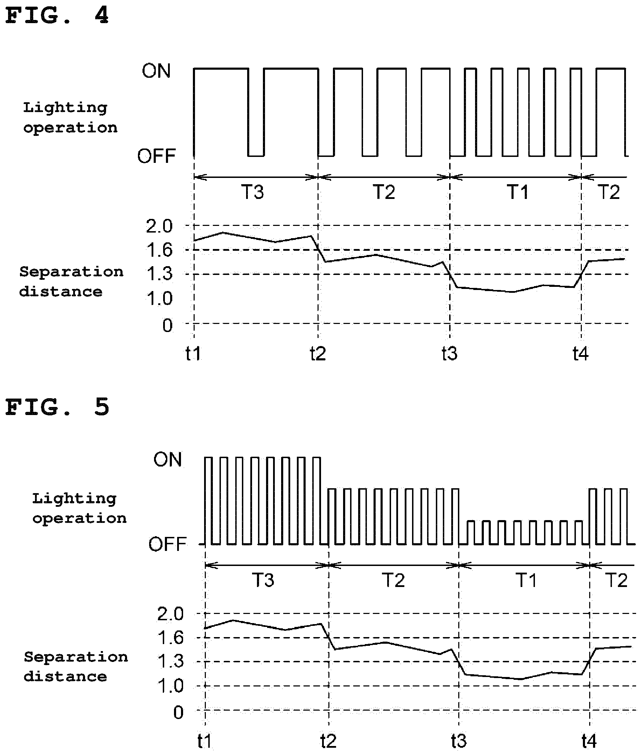

FIG. 4 is a timing chart illustrating an example of an operation in which the lighting duty ratio is changed by changing the lighting time of intermittent lighting based on the signal from the distance sensor 32. FIG. 4 shows a conceptual diagram, and the lighting (ON) time and non-lighting (OFF) time are different from those shown in the above-mentioned Table 3. In this case, the controller 16 in the ultraviolet light irradiation device 100 executes the lighting operation pattern of the operation mode 3 in Table 3 during the period T3 (time t1 to t2). Then, during the period T2 (time t2 to t3), the controller 16 reduces the lighting duty ratio of intermittent lighting by shortening the lighting time that that of the period T3. Furthermore, during the period T1 (time t3 to t4), the controller 16 reduces the lighting duty ratio of intermittent lighting by shortening the lighting time further than that of the period T2.

In the above embodiments, described are the cases in which the lighting duty ratio is variably controlled by changing either the lighting time or the non-lighting time; however, there may be the case in which both the lighting time and the non-lighting time are changed. Not only this, but the amount of ultraviolet light emitted in accordance with a given section may also be controlled by changing the illuminance of ultraviolet light on the light emission surface 12 (Table 4). Methods of changing the illuminance of ultraviolet light on the light emission surface 12 include adjusting the voltage applied to the light-emitting element (excimer lamp) in the light source section, adjusting the frequency of the voltage applied to the light-emitting element (excimer lamp) in the light source section.

**Table 4**

| Name | Separation distance from light emission surface | Lighting operation |
|---|---|---|
| | Section (Distance range) [m] | Illuminance [mW/cm²] |
| Operation mode 1 | 1.0-1.29 | 10 |
| Operation mode 2 | 1.3 - 1.59 | 20 |
| Operation mode 3 | 1.6 - 1.99 | 35 |
| Operation mode 4 | 2.0 or more | 55 |

The illuminance shown in Table 4 is the illuminance of ultraviolet light at a position, for example, 20 mm away from the light emission surface 12 in a direction perpendicular to the light emission surface 12. Here, the illuminance shown in Table 4 is set such that the illuminance of ultraviolet light at the irradiated surface (the surface irradiated with the ultraviolet light) is nearly the same in each section.

FIG. 5 is a timing chart illustrating an example of an operation in which the amount of ultraviolet light is changed by changing the illuminance of the intermittent lighting operation based on the signal from the distance sensor 32. Here, the lighting duty ratio of the intermittent lighting is constant. The vertical axis of FIG. 5A shows the lighting operation of ON/OFF and the illuminance at the light emission surface 12 during the lighting (ON) time. FIG. 5 is a conceptual diagram, and the values of illuminance in each mode are different from those of illuminance shown in the above-mentioned Table 4. In this case, the controller 16 of the ultraviolet light irradiation device 100 executes the lighting operation of the operation mode 3 in Table 4 during the period T3 (time t1 to t2). Then, the controller 16 controls the intermittent lighting at lower illuminance during the period T2 (time t2 to t3) than that during the period T3. Furthermore, the controller 16 controls the intermittent lighting at even lower illuminance during period T1 (time t3 to t4) than that period T2.

In order to further reduce the amount of ultraviolet light, the reduction of illuminance illustrated in FIG. 5 may be executed in conjunction with the reduction of the lighting duty ratio illustrated in FIG. 3 and FIG. 4. In the above embodiments, described are the cases in which the ultraviolet light irradiation mode from the light source section is an intermittent lighting mode; however, a continuous lighting mode in which the ultraviolet light irradiation is executed continuously from the light source section can also be adopted. In this case, the controller 16 of the ultraviolet light irradiation device 100 changes the illuminance at the light emission surface 12 of the ultraviolet light emitted from the light source section based on the signal from the distance sensor 32.

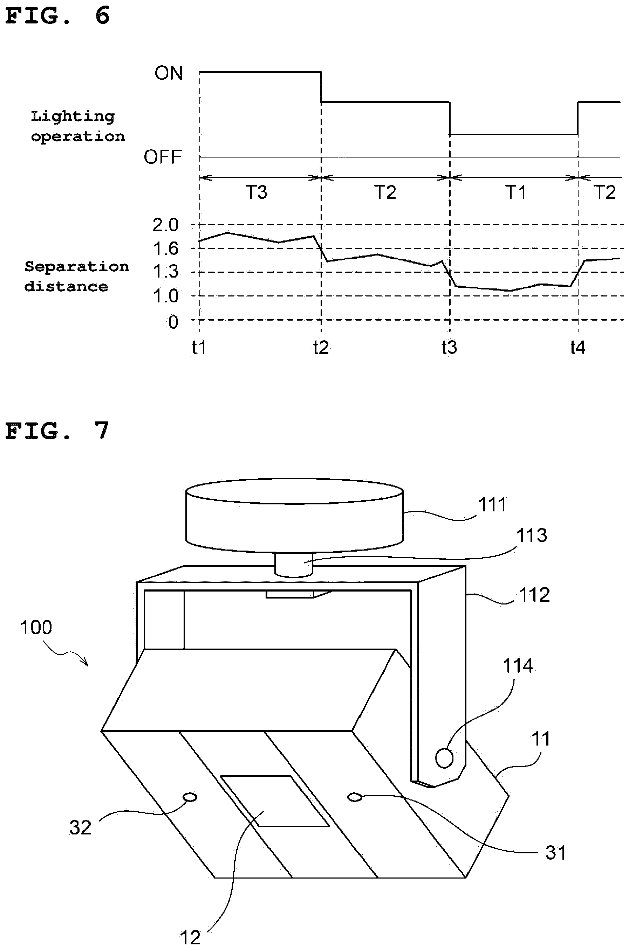

FIG. 6 is a timing chart illustrating an example of an operation in which the amount of ultraviolet light is changed by changing the illuminance of the continuous lighting operation based on the signal from the distance sensor 32. Here, the vertical axis in FIG. 6A shows the lighting operation of ON/OFF and the illuminance at the light emission surface 12 during the lighting (ON) time. In this case, the controller 16 of the ultraviolet light irradiation device 100 executes the lighting operation of the operation mode 3 in the period T3 (time t1 to t2). Then, the controller 16 controls the continuous lighting at lower illuminance during the period T2 (time t2 to t3) than that during the period T3. Furthermore, the controller 16 controls the continuous lighting at even lower illuminance during the period T1 (time t3 to t4) than the period T2.

In the above embodiments, it is first assumed that setting a predetermined distance range in the section (distance range), which is set in terms of the separation distance from the light emission surface 12, prevents the lighting operation mode from overreacting due to the movement of a moving object (including a human and an object) when a moving object enters the space. For example, setting a predetermined distance range in the section (distance range) prevents the lighting operation mode from reacting unnecessarily with a movement of a person standing up in a chair, stretching, and moving his/her neck from side to side, for example. The distance range of this section is set appropriately in accordance with the location at which the device is used and the use cases therefor. For example, the distance range can be suitably changed from 10 cm to 2 m.

The above embodiments describe a case in which a pyroelectric human detection sensor is used as the detector 31, but the detector 31 is not limited to this type. The detector 31 can be configured, for example, such that a reflector may be installed at a position (e.g., on a wall, a floor, a desk, etc.) in the space where the ultraviolet light irradiation device 100 is disposed, and the detector 31 emits light toward the reflector and detects the presence of a person in the space by whether the light reflected by the reflector is detected or not. In this case, the detector 31 determines that a person is present in the space when the reflected light from the reflector fails to be detected.

In addition, in the above embodiment, described is the case in which the orientation of the light emission surface 12 is fixed; however, the ultraviolet light irradiation device 100 may be provided with a drive unit that changes the orientation of the light emission surface 12. In this case, the distance sensor 32 measures the separation distance to an object facing the light emission surface 12 in response to the orientation of the light emission surface 12. For example, the ultraviolet light irradiation device 100 is provided with a light source section having a swiveling function, and emits ultraviolet light over a wide area while changing the position of the ultraviolet light irradiation by changing the swinging angle in a predetermined pattern or in a pattern instructed by the user.

FIG. 7 is a schematic view of an example of an ultraviolet light irradiation device 100 capable of radiating ultraviolet light in a variable direction. As shown in FIG. 7, the ultraviolet light irradiation device 100 is supported by a supporter 112 fixed to a mounting portion 111 that is to be attached to a ceiling, for example. Here, the supporter 112 is fixed to the mounting portion 111 in a rotatable manner around a rotation shaft 113. The ultraviolet light irradiation device 100 is also rotatably supported by the supporter 112 around a rotation axis 114. This configuration allows the ultraviolet light irradiation device 100 to swivel up and down, left and right. The configuration of the ultraviolet light irradiation device 100 having a swiveling function is not limited to the configuration shown in FIG. 7.

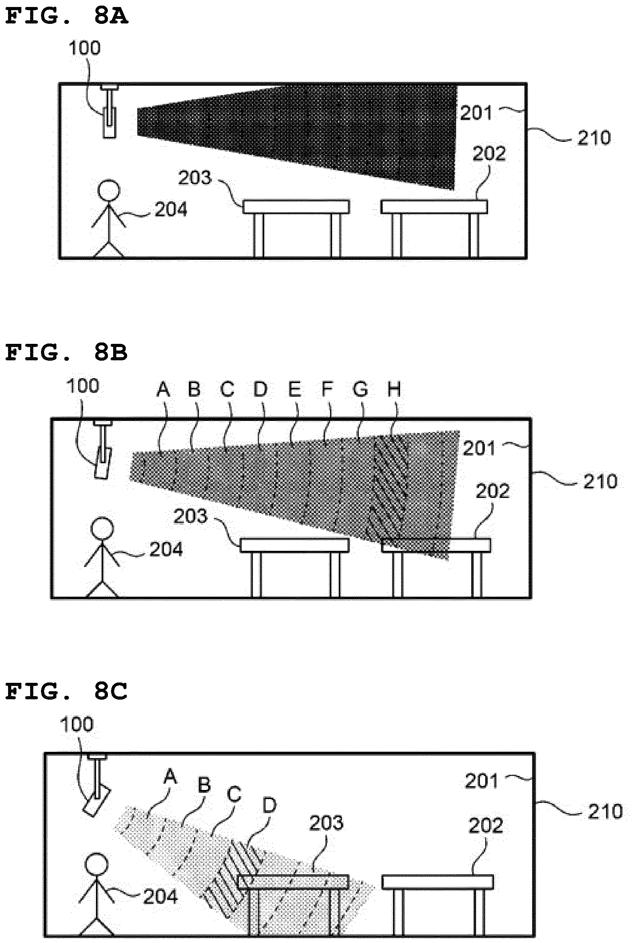

FIGS. 8A to 8D are schematic views illustrating the case where the ultraviolet light irradiation device 100 swivels in an angle range of 90 degrees in the up-down direction. The ultraviolet light irradiation device 100 is disposed in a space 210 defined by a wall 201, and desks 202 and 203 are placed and a person 204 is present in the space 210. As shown in FIG. 8A, if the ultraviolet light irradiation device 100 has the light emission surface 12 (not shown in FIG. 8A) facing the horizontal direction, the controller 16 determines, based on the signal from the distance sensor 32, that an object facing the light emission surface 12 are sufficiently far apart, and emits a relatively large amount of ultraviolet light from the light source section. This enables powerful disinfection and inactivation in the space 210.

Then, as shown in FIG. 8B, when the ultraviolet light irradiation device 100 is made to turn the light emission surface 12 (not shown in FIG. 8B) slightly downward from the horizontal direction, the distance sensor 32 detects the desk 202 in a section H that is relatively far from the light emission surface 12. This allows the selection of the operation mode corresponding to the distance from the light emission surface 12 to the desk 202, thereby reducing the amount of ultraviolet light emitted from the light source section compared to the state shown in FIG. 8A. After that, as shown in FIG. 8C, when the ultraviolet light irradiation device 100 is made to turn the light emission surface 12 (not shown in FIG. 8C) further downward, the distance sensor 32 detects the desk 203 in the section D that is relatively close to the light emission surface 12. This allows the selection of the operation mode corresponding to the distance from the light emission surface 12 to the desk 203, thereby reducing the amount of ultraviolet light emitted from the light source section compared to the state shown in FIG. 8B.

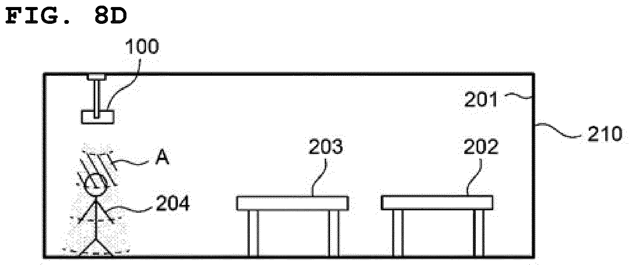

If an object (in this case, the desk 203 made of resin) is exposed to strong ultraviolet light, the front surface of the desk 203 may be degraded due to the ultraviolet light. Reducing the amount of ultraviolet light emitted from the light source section as described above makes it possible to suppress the desk 203 located at a relatively close distance from the light emission surface 12 from being irradiated with the strong ultraviolet light, thereby suppressing the degradation of the surface of the desk 203. After that, as shown in FIG. 8D, when the ultraviolet light irradiation device 100 is made to turn the light emission surface 12 (not shown in FIG. 8D) downward in the vertical direction, the distance sensor 32 detects the person 204 in the section A that is closest to the light emission surface 12. This allows the selection of the operation mode corresponding to the distance from the light emission surface 12 to the person 204, thereby reducing the amount of ultraviolet light emitted from the light source section to the lowest level. Accordingly, even when the orientation of the light emission surface 12 changes, the separation distance between the light emission surface 12 and an object can be appropriately measured so as to control to switch to a suitable lighting operation mode.

The case where the swinging angle is changed in a predetermined pattern or in a pattern instructed by a user has been described; however, the ultraviolet light irradiation may be performed with fixing a swinging angle at an angle instructed by a user. For example, a user can use a remote controller, a smartphone, or other devices to instruct a swinging angle such that ultraviolet light radiates to an area where disinfection and inactivation are desired to be intensively executed. In this case, the separation distance between the light emission surface 12 and the object can be measured at a swinging angle instructed by a user, thereby enabling the operation in the suitable lighting operation mode.

The amount of the ultraviolet light in each lighting operation mode can be determined by whether the "cumulative amount of ultraviolet light radiation per unit time" is relatively low or not. When the light source section is subject to a periodic lighting control by the controller 16, the "cumulative amount of ultraviolet light radiation per unit time" is referred to as the value such that the cumulative amount of ultraviolet light radiation in each lighting period (one period) is divided by the time of the lighting period. For example, when a lighting period consists of a lighting time (ON time) and a non-lighting time (OFF time), it is the value such that the cumulative amount of ultraviolet light radiation emitted during the lighting period is divided by the sum of the lighting time and the non-lighting time. In other words, the "cumulative amount of ultraviolet light radiation per unit time" corresponds to an average value of the cumulative amount of radiation during each lighting period.

When the light source section is not subject to a periodic lighting control by the controller 16 and is configured to be such that a periodic lighting control is impossible, for example, when only continuous lighting control is executed during the lighting, the "cumulative amount of ultraviolet light radiation per unit time" is set to be the value such that the cumulative amount of ultraviolet light radiation during any predetermined time (i.e., 5 minutes, 10 minutes) is divided by the predetermined time (i.e., 5 minutes, 10 minutes in the example above). In this case, the "cumulative amount of ultraviolet light radiation per unit time" corresponds to an average value of the cumulative amount of radiation during the continuous lighting operation.

### Reference Signs List

- 11: Housing
- 12: Light emission surface
- 15: Power supply section
- 16: Controller
- 20: Ultraviolet light source
- 21: discharge container
- 22: first electrode
- 23: second electrode
- 31: Detector
- 32: Distance sensor
- 100: Ultraviolet light irradiation device
- 200: Object

## Claims

1. An ultraviolet light irradiation device comprising:
a light source section having a light emission surface emitting ultraviolet light having a wavelength band from 190 nm to 235 nm;
a controller that controls lighting of the light source section; and
a distance sensor that measures a separation distance from the light emission surface to an object facing the light emission surface,
wherein the controller is provided with a plurality of lighting operation modes and controls to switch the lighting operation mode based on a signal from the distance sensor, and
each of the plurality of lighting operation modes being determined corresponding to a section defined by the separation distance, and being set such that an amount of the ultraviolet light emitted from the light source section differs from each other.

2. The ultraviolet light irradiation device according to claim 1, wherein the plurality of lighting operation modes is set such that the amount of the ultraviolet light emitted from the light source section becomes lower in the section to which the separation distance from the light emission surface is relatively closer.

3. The ultraviolet light irradiation device according to claim 2, wherein the plurality of lighting operation modes is set such that the illuminance of the ultraviolet light emitted from the light source section at the light emission surface becomes lower in the section to which the separation distance from the light emission surface is relatively closer.

4. The ultraviolet light irradiation device according to claim 2, wherein the plurality of lighting operation modes controls the lighting of the light source section such that a lighting duty ratio of the light source section is reduced in the section to which the separation distance from the light emission surface is relatively closer.

5. The ultraviolet light irradiation device according to any one of claims 1 to 4, further comprising a human detection sensor that detects a location of a person facing the light emission surface,
wherein the controller controls to switch the lighting operation mode based on a signal from the distance sensor when a presence of a person is detected by the human detection sensor.

6. The ultraviolet light irradiation device according to claim 5, wherein the controller is set such that when the presence of a person is undetected by the human detection sensor, the amount of ultraviolet light emitted from the light source section becomes higher than that when the presence of a person is detected by the human detection sensor.

7. The ultraviolet light irradiation device according to any one of claims 1 to 4, further comprising a drive unit that changes an orientation of the light emission surface,
wherein the distance sensor measures the separation distance to an object facing the light emission surface corresponding to the orientation of the light emission surface.

8. An ultraviolet light irradiation method of controlling lighting of a light source section having a light emission surface emitting ultraviolet light having a wavelength band from 190 nm to 235 nm, the ultraviolet light irradiation method comprising the steps of:
receiving a signal from a distance sensor measuring a separation distance to an object facing the light emission surface; and
switching a plurality of lighting operation modes to control the lighting of the light source section based on the signal from the distance sensor, each of the plurality of lighting operation modes being determined corresponding to a section defined by the separation distance from the light emission surface, and being set in a manner that an amount of the ultraviolet light emitted from the light source section is different from each other.
